# EUROPEAN PATENT APPLICATION

(11) **EP 4 480 500 A1**
(43) Date of publication of application: **25.12.2024**
(21) Application number: 23180730.6
(22) Date of filing: 21.06.2023
(51) Int. Cl.: A61K 47/69, C12N 5/00, C12N 5/078

(54) **MODIFIED CELL, PHARMACEUTICAL COMPOSITION AND METHOD OF PREPARING A MODIFIED CELL**

(71) Applicant: Max-Planck-Gesellschaft zur Förderung der Wissenschaften e.V., 80539 München (DE)
(72) Inventor: Hernandez Bücher, Jochen Estebano, 69123 Heidelberg (DE); Plazman, Yilia, 70195 Stuttgart (DE); Spatz, Joachim, 70569 Stuttgart (DE)
(74) Representative: Manitz Finsterwald Patent- und Rechtsanwaltspartnerschaft mbB

(57) **Abstract**

The present invention relates to a modified cell, in particular a modified blood cell, having a cell membrane encapsulating a lumen of the modified cell, wherein a functional lipid is incorporated into the cell membrane, wherein the functional lipid has a bonding site capable of bonding to a molecule having a functional motif, and wherein the bonding site is located on an external surface of the cell membrane. Further, the present invention concerns a pharmaceutical composition, the use of the modified cell and/or the pharmaceutical composition for use as a medicament or as a contrast agent as well as a method of modifying a cell.

## Description

The present invention relates to a modified cell, a pharmaceutical composition comprising such a modified cell, the use thereof as a medicament or contrasting agent as well as to a method of surface modifying a cell.

The method of the present invention is capable of synthetic modification natural cells, in particular of ghost red blood cells (GRBCs) or vesicle-like counterparts which are obtainable by extrusion, comprising at least two lipids and at least two immune cell associated proteins. The synthetic modification of natural cells, such as GRBCs, is based on the incorporation of functional lipids into the GRBC membrane with sequential bottom-up engineering of target specific cytotoxic immune cells by the immobilizing of recombinant proteins. The inventive method allows producing of surface modified natural cells, in particular of surface modified GRBC (smGRBC), with new biological functions. Such modified cells with specific lipids and proteins as functional motifs are described herein, as well as their therapeutic use.

The immune system is an effective barrier against intruding pathogens and a bulwark against malignant cells. The basis for this is an adaptive, target specific and fast clearance system. Hereby, a self-organized interplay between individual immune elements orchestrates an intricate and complex network between cellular and molecular processor and effector units [1]. However, some infected or transformed cancerous cells are able to undergo this effective bulwark causing severe damage and eventually cancer [2]. As an advanced therapeutic option, the CAR-T technology has been developed as a promising immunotherapy approach combining the immune system unmatched specificity and cytotoxicity. This technology is based on the genetic modification of lymphocytes such as T-cells by introducing an engineered chimeric antigen receptor on the cell surface, giving them the ability to target and kill malignant cells [3,4]. However, this top-down approach has some limitations in terms of versatility, off-target side effects and a long-winding personalization process thus making it still a cost inefficient technology [5]. Encouraged by this approach, a bottom-up synthetic biology approach was used to assemble an immune inspired synthetic cell (iisC). Herby, a synthetic cell surrogate which is able to induce apoptosis in target cells and at the same time to discriminate between two cell lines was developed [6]. However, therapeutic approaches based on synthetic cells are limited in their applicability as they are very fast removed by the immune clearance system when introduced intravenously [7-9]. This diminishes their therapeutic potential dramatically.

In view of the above, the problem underlying the present invention is to overcome the disadvantages of CAR-T technology and bottom-up synthetic biology approaches.

The present invention solves this problem by providing a modified cell according to claim 1, a pharmaceutical composition according to claim 21 and a method of modifying a cell according to claim 26.

In accordance with the present invention, the modified cell, in particular a modified blood cell is a cell having a cell membrane encapsulating a lumen of the modified cell, wherein a functional lipid is incorporated into the cell membrane, wherein the functional lipid has a bonding site capable of bonding to a molecule having a functional motif, and wherein the bonding site is located on an external surface of the cell membrane. Further, the present invention relates to a pharmaceutical composition comprising a modified cell according to present invention.

It was found that such a modified cell can be easily functionalized via the bonding site. Thereby, a high versatility is achieved. Based on the modified cell of the present invention, it is not required to engage in a long-winded personalization process. Further, it was found that the modified cell of the present invention, i.e. a modified natural cell which is a natural cell that has been modified, remains in the bloodstream for a long time in the blood, i.e. is not removed by the immune clearance system. It is believed that the reason for this is that the modified natural cell does not appear as a foreign matter to the immune system since it has, apart from the functional lipid with the bonding site, preserved its natural cell membrane including e.g. membrane proteins and glycolipids. Therefore, the modified cell according to the present invention appears natural to an immune system which is not the case for an immune inspired synthetic cell (iisC). The modified cell of the present invention can also be referred to as a surface modified cell, since the functional lipid provides the bonding site on the surface of the modified cell.

Further, the present invention relates to a method of modifying a cell, wherein the method comprises the steps of (A) providing a target cell to be modified, (B) incorporating a functional lipid into the cell membrane of the target cell, wherein the functional lipid has a bonding site, and optionally (C) linking a functional motif to the binding site of the lipid.

By incorporating the functional lipid into the cell membrane, a bonding site is provided on the cell surface. Thereby, it is possible to produce a modified cell in accordance with the present invention.

In a preferred embodiment, the modified cell is a modified natural cell. As set out above, a natural cell has specific surface characteristics, such as membrane proteins and glycolipids that can be recognized by the immune system. Therefore, by modifying a natural cell, i.e. not a synthetic cell, the modified cell still has these surface characteristics and can circulate in the bloodstream much longer compared to synthetic cells which are rapidly cleansed by a body's immune system. The natural cell can be a ghost natural cell, i.e. a natural cell from which specific components, e.g. hemoglobin, have been removed.

It is particularly preferred that the modified cell is a modified blood cell, in particular a modified red blood cell (erythrocyte) or a modified platelet (thrombocyte), in particular a modified ghost red blood cell. Blood cells are by nature circulating in a blood stream. Average life span differs greatly between these cell types. In physiological conditions red blood cells (RBCs) have a life span of around 100 to 120 days, making them a perfect delivery platform of drugs and bioactive components, whereas platelets have a rather short life span of 8 to 9 days in average [10, 11]. The cell type may be chosen based on the kind of treatment or effect that is desired. In case the treatment should be long lasting, e.g. for treating a tumor disease, red blood cells or ghost red blood cells appear advantageous. In case the treatment or effect is only desired for a short time, e.g. for labeling during radiology, it may be advantageous to use platelets instead.

Ghost red blood cells (GRBCs) are natural membrane shells produced from RBCs. GRBCs are produced by hemolysis of erythrocytes leaving an empty membrane shell behind. During hemolysis major biological properties of red blood cells for a prolonged circulation remain intact. In addition, the GRBC' s lumen can be loaded with large amounts of drugs and other pharmaceutic active components and several attempts have been made to reconceptualize their biophysical properties of the cell surface have been reported [12-14].

In the examples described below, surface modified GRBCs (smGRBCs) are designed to rebuild immune cell properties in terms of specificity and cytotoxicity in a controlled and cost-efficient manner. The smGRBCs as designed in this invention may serve as on demand biomedical tools for various *in vivo* and *in vitro* therapeutic and diagnostic applications, such as target specific anti-tumor therapy, immune-modulatory therapies such as adaptive based cell therapy, pathogen vaccination and drug delivery.

The examples described below demonstrate a method for the surface modification of GRBCs providing them with analogous and combined properties of natural killer, (NK), T and B-cells. The smGRBCs are equipped with the prolonged circulation properties of its natural counterpart as well as the cytotoxic and specific immunological functionalities. The method is based on engineering the surface of GRBCs by incorporating functional lipids into the membrane. The incorporation of functional lipids refers to the process in which single lipids are integrating the hydrophobic part into the outer membrane leaflet. Single lipids are obtained by dissolving them in a solution containing fatty free bovine serum albumin. During this process micelle formation is prevented and single lipids remain in the solution.

The functional lipid of the modified cell in accordance with the present invention is preferably bonded to a molecule having a functional motif *via* the bonding site. By bonding to the functional motif, the modified cell can be programmed with a desired functionality, such as apoptosis generation capabilities on cancer cells. However, even before binding the functional motif, the modified cell of the present invention is useful as a template that can be functionalized.

The bonding of the functional motif can be achieved by different means, such as by formation of a covalent bond or by complex formation. In accordance with a preferred embodiment of the present invention, the functionalization, i.e. the bonding of the functional motif, is achieved by complex bond formation between the functional lipid and a molecule carrying the functional motif. In accordance with another preferred embodiment of the present invention, the functionalization, i.e. the bonding of the functional motif, is achieved by forming a covalent bond, e.g. by means of a click chemistry reaction, between the functional lipid and a molecule carrying the functional motif.

Preferably, the bond between the functional lipid and the functional motif is obtained by formation of a complex, wherein the complex comprises (i) a metal cation, (ii) the bonding site of the functional lipid, and (iii) a molecule with the functional motif, wherein the bonding site of the functional lipid and the molecule with the functional motif are ligands to the metal cation. More preferably, the bonding site of the functional lipid is a chelating ligand.

Preferably, the bond formation is realized by complex formation as described in WO 2020/254539 A1, the entire content of which is herewith incorporated by reference.

Preferably, the bonding site of the ligand can act as a lewis base and has 2,3, 4, 5 or more chemical groups that are capable of coordinating to a metal cation. More preferably, the synthetic ligand has a chemical structure derived from nitrilotriacetic acid (NTA), iminodiacetic acid (IDA), tris(carboxymethyl)ethylenediamine (TED), triazacyclononane (TACN), diethylenetriamine-pentaacetate (DTPA), phytochelatin, carboxymethylaspartate (CMA), phosphonates, tannic acid (TA), porphyrin, dipyridylamine (DPA), phytic acid, nitrilopropionicdiacetic acid (NPDA), nitriloisopropionicdiacetic acid (NIPDA), N-(hydroxylethyl)ethylenediaminetriacetic acid (HEDTA), 1,4,7,10-tetraazacyclodo-decane-N,N',N",N‴-tetraacetic acid (DOTA), 1,4,7-tris(carboxymethyl)-10-(2'-hydroxypropyl)-1,4,7,10-tetraazocy-clodecane, 1,4,7-triazacyclonane-1,4,7-triacetic acid (NOTA), 1-(1,3-carboxypropyl)-1,4,7- triazacyclononane-4.7-diacetic acid (NODAGA), 1,4,8,11 -tetraazacy-clotetra-decane- N,N',N",N‴-tetraacetic acid (TETA), ethylenedicysteine, ethylenediaminetetraacetic acid (EDTA), 1,2-diaminocyclohexane-N,N,N',N'-tetraacetic acid (DACT), bis(aminoethanethiol)carboxylic acid, ethylene-bis(oxyethylene-ni-trilo)tetraacetic acid (EGTA), triethylenetetramine-hexaacetic acid (TTHA), 1,4,7-triazacyclononane phosphinic acid (TRAP), deoxyribonucleic acid (DNA) and ribonucleic acid (RNA), purine, and pyridimidine and derivatives thereof. The terminology "chemical structure derived from" indicates that the molecular structure mentioned as the chemical structure is bonded to the functional lipid by a covalent bond requiring e.g. in case of EDTA one of the hydrogens of the CH₂-groups of the ethylene-fragment may be a covalent bond to the rest of the functional lipid.

More preferably, the bonding site of the functional ligand has a chemical structure derived from nitrilotriacetic acid (NTA), iminodiacetic acid (IDA), chelating peptides with the consensus sequence (GHHPH) n G, diethylenetriamine- pentaacetate (DTPA), nitrilopropionicdiacetic acid (NPDA), nitriloisopropionicdiacetic acid (NIPDA), ethylenediamine-tetraacetic acid (EDTA), ethylene-bis(oxyethylene- ni-trilo)tetraacetic acid (EGTA), carboxymethylaspartate (CMA) and derivatives thereof.

Even more preferably, the bonding site of the functional ligand has a chemical structure derived from NTA, IDA and derivatives thereof. NTA and IDA are tetradentate and tridentate ligands, respectively. Accordingly, both bind particularly strong to metal cations. Since IDA is a tridentate ligand, it binds less strong compared to NTA, however complex formation with IDA may be faster than with NTA. Strong binding to the metal cation, as by NTA or IDA derived structures, prevents release of the metal cation and of the molecular fragment with the functional motif. Thereby, the modified cell functions efficiently as carrier for the functional motif.

As mentioned herein, a "derivative" is a compound having the same lead structure, but may be substituted by further chemically reactive groups. Preferably, a "derivative" has a substitution of the lead structure with one or more additional chemical groups selected from the group consisting of carboxyl, amine, azide, acrylate, maleimide, hydroxyl, thiol, aromatic, aliphatic, disulfate, and vinylsulfone groups. The term derivative includes also molecules or molecular fragments with isotopic substitutions. "Isotopic substitution" means that one or more of the atoms are isotope labeled.

Preferably, the metal cation of the complex is a di-, tri- or tetravalent metal cation. Suitable examples of the metal cation are metal cations selected from the group consisting of: Ni²⁺, Co³⁺, Cr³⁺, Rh³⁺, Ir³⁺, Pt²⁺, Pt⁴⁺, Ru²⁺, Ru³⁺, La³⁺, Eu³⁺, Os²⁺, Pd⁴⁺, Mo³⁺, Fe³⁺, Ru³⁺, Gd³⁺, Tc³⁺, Re³⁺, Sm³⁺, Tb³⁺, Ce³⁺, Pr³⁺, Nd³⁺, Pm³⁺, Dy³⁺, Ho³⁺, Er³⁺, Tm³⁺, Yb³⁺, V²⁺, Mn⁴⁺, Fe²⁺ and Lu³⁺. Preferably, the metal cation is selected from the group consisting of: Ni²⁺, Co³⁺, Cr³⁺, Rh³⁺, Ir³⁺, Ir⁴⁺, Pt²⁺, Pt⁴⁺, Pd⁴⁺, Mo³⁺, Fe³⁺, Gd³⁺, Tb³⁺, Eu³⁺, Ru²⁺, La³⁺, Ru³⁺, Re³⁺, Re⁴⁺, V²⁺, Mn⁴⁺, Fe²⁺ and Os²⁺. More preferably, the metal cation is Ni²⁺ or Co³⁺. Most preferably, the metal cation is Co³⁺.

According to a preferred embodiment, the molecule having the functional motif is capable of forming a complex with a metal cation, such as those mentioned above, and the part of the functional lipid that acts as a ligand to the metal cation as well.

According to a preferred embodiment, the molecule having the functional motif has an affinity tag. Preferably, the affinity tag is a His-tag having six to nine histidine moieties. More preferably, the affinity tag is a chelate ligand to the metal cation.

While it is preferred that the molecule having the functional motif is bonded to the functional lipid via complex formation, such as described in WO 2020/254539 A1, the present invention is not limited thereto. According to an alternative embodiment, the functional motif is bonded to the functional lipid *via* a covalent bond. The covalent bond may be created by chemical reaction between a reactive group at the functional lipid, i.e. the reactive group is the bonding site, and another group provided at the molecule having the functional motif. Such a reaction can be selected from a wide variety of reactions. Click chemistry reactions are suitable examples for such reactions. Exemplary click chemistry reactions are azide alkyne cycloadditions, such as the Copper(I)-catalyzed azide-alkyne cycloaddition (Cu-AAC), strain-promoted azide-alkyne cycloaddition (SPAAC) and strain-promoted alkyne-nitrone cycloaddition (SPANC), thiol-ene reactions, and other reactions commonly known as click reactions. For some applications, metal free click reactions can be preferred.

The functional lipid is not particularly limited, as long as it can be incorporated into the cell membrane of a cell to be surface modified. It is preferable that the functional lipid is a polar lipid, in particular preferably a phospholipid. In addition to DGS-NTA lipids the functional lipid may alternatively be cholesterol-based, a sulfolipid, a glycolipid, DNA functionalized lipids, Sortase-tagged lipids, biotinylated lipids, NHS functionalized lipids or a mixture of these lipids.

As demonstrated by the examples below, suitable functional lipids are for example 1'2-Dioleoyl-sn-glycero-3-phosphoethanolamin, Atto488 and Atto647 conjugated as well as 1 '2-dioleoyl-sn-glycero-3- [(N.-(5 -amino- 1 -carboxypentyl)iminodiacetic acid) succinyl] (nickel salt) DGS-NTA(Ni)²⁺ and DGS-NTA (Co)³⁺ were incorporated inside ghost red blood cell membrane.

### [functional motif]

It is not particularly limited what kind of functional motif is bonded to the bonding site of the modified cell of the present invention. In a preferred embodiment, the functional motif is based on a protein, in particular wherein the molecule with the functional molecule is a recombinant protein combining the functionality of the protein and is provided with a site to bond to the bonding site of the functional lipid, a label or a contrasting agent. Alternative preferred functional motifs, it can be metal nanoparticles, streptavidin and/or biotinylated proteins, complementary DNA and/or DNA functionalized proteins, molecules and proteins with Sortase recognition sequences, proteins with terminal amine groups and a mixture of these motifs.

The terminology "based on a protein" indicates that functionalities of a natural protein on which the functional motif is based on provided by the functional motif itself. Preferably, this is realized by a recombinant protein having the natural amino acid sequence of the protein and preferably an affinity tag on one end of the amino acid sequence.

In a preferred embodiment of the present invention, the functional motif bonded to the bonding site of the functional lipid in the cell membrane of the modified cell is a protein. It is particularly preferred that the functional motif is based on a protein from the tumor necrosis factor superfamily. Most preferably, the functional motif is at least one of FasL (synonyms are CD95L or CD178) and Trail (synonyms are CD253, APO-2L). It was found that such tumor necrosis factors can induce apoptosis highly efficient in tumor cells when being bonded to the surface of a modified cell.

According to another embodiment of the present invention, the functional motif is a label, in particular a fluorescence label, radioactive label and magnetic label.

The modified cell according to the present invention and the pharmaceutical composition of the present invention are particularly suitable for use as a medicament or as a contrast agent. Contrast agents are useful for imaging technology such as diagnostic imaging. By administering them via surface modified cells according to the present invention, it can be possible to deliver the contrast agents directly to locations of interest, provided that the surface modified cells are provided with target specific functional motifs. In a preferred embodiment of the present invention, the modified cell or the pharmaceutical composition are for use as a medicament, in particular as a medicament which is administered by injection, in particular by injection into a vein. According to a preferred embodiment of the present invention, the pharmaceutical composition is for use in the treatment of a disease, selected from the group consisting of tumor diseases, cardiovascular disease, neurodegenerative diseases, autoimmune diseases, and inflammation diseases.

In a particularly preferred embodiment of the present invention, the modified cell and/or pharmaceutical composition of the present invention are for use in an anti-tumor therapy, in immune-modulatory therapies such as adaptive based cell therapy, pathogen vaccination and drug delivery. In a preferred embodiment, the modified cell and/or pharmaceutical composition of the present invention are for use in in the treatment of a tumor disease. The tumor disease is in particular one selected from the group consisting of leukemia, carcinoma, sarcoma and neuroectodermal cancers.

The method according to the present invention preferably provides a modified cell in accordance with the present invention. Preferably, the modified cell according to the present invention is a modified cell obtainable by the method according to the present invention.

In the following the invention is described by representative non-limiting examples and figures. In the figures the following is shown:
- Fig. 1a: Fluorescence microscope image showing functionalization of the cell membrane of a surface modified ghost red blood cell in accordance with the present invention with Atto647-conjugated DOPE lipids.
- Fig. 1b: Fluorescence microscope image showing functionalization of the cell membrane of a surface modified ghost red blood cell in accordance with the present invention with Atto488-conjugated DOPE lipids.
- Fig. 2a: Incorporation of DGS-NTA(Co)³⁺ into the membrane of human GRBCs and immobilization of recombinant 6x His-tagged green fluorescent protein. Left column shows bright field images of human GRBCs. Right column shows staining for recombinant 6x His-tagged green fluorescent protein indicated as membrane staining.
- Fig. 2b: Incorporation of DGS-NTA(Co)³⁺ into the membrane of mice GRBCs and immobilization of recombinant 6x His-tagged green fluorescent protein. Left column shows bright field images of mice GRBCs. Right column shows staining for recombinant 6x His-tagged green fluorescent protein indicated as membrane staining.
- Fig. 3a: Cytotoxic potential of FasL- and Trail-functionalized smGRBCs as verified by flow cytometry.
- Fig. 3b: Amount of apoptotic positive cells determined by Annexin V staining.
- Fig. 3c: Microscope images showing caspase-8 activation.
- Fig. 3d: Quantification of the percentage of cleaved caspase-8 positive cells.
- Fig. 4: Confocal laser scanning microscopy images of smGRBCs showing immobilization of Fc-receptors (Protein G) and immunoglobulins conjugated to an Alexa555 fluorophor.
- Fig. 5: Fluorescent confocal microscopy images of WBC264-9C macrophages stained with LysoTracker red (lyososmes, red), incubated with Alexa488-TFP stained GRBCs or smGRBCs (plasma membrane, green) or Giant Unilamellar Vesicles (GUVs).
- Fig. 6a: Examination of cytotoxic potential of mice smGRBC immobilized with cytotoxic ligands according to the present invention and controls on a human cancer cell line.
- Fig. 6b: Examination of cytotoxic potential of mice smGRBC immobilized with cytotoxic ligands according to the present invention and controls on a mice cancer cell line.
- Fig. 7: Flow cytometric quantification of PI positive Jurkat T-cells incubated with modified cells according to the present invention and comparative test results.
- Fig. 8: Head-to-head comparison of human smGRBCs complexed with (Ni)²⁺ or (Co)³⁺ (modified cells according to the present invention).

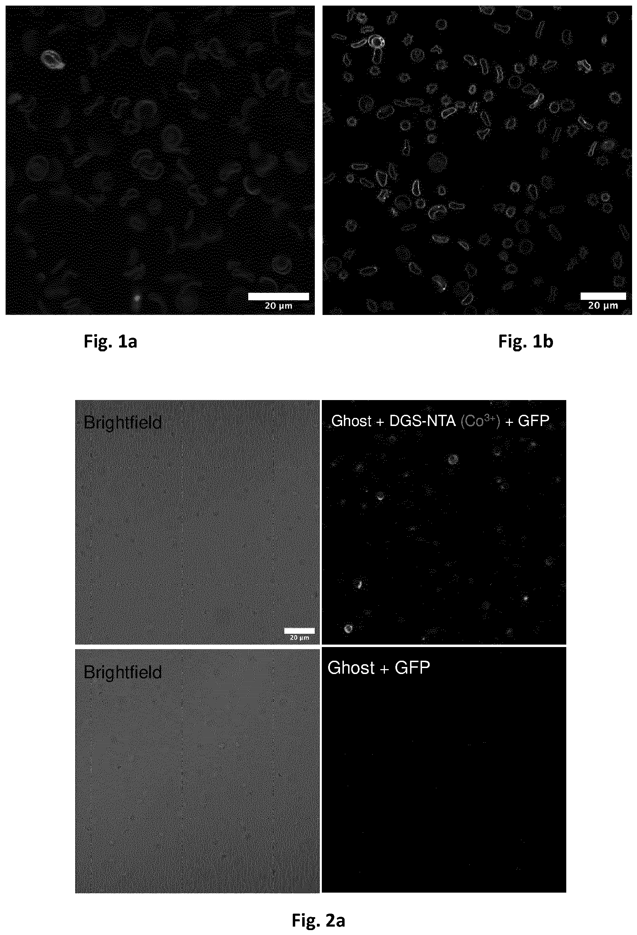

To demonstrate the ability to incorporate a functional lipid into a cell membrane, 1'2-Dioleoyl-sn-glycero-3-phosphoethanolamin, Atto488 and Atto647 conjugated, as well as 1'2-dioleoyl-sn-glycero-3- [(N.-(5 -amino- 1 -carboxypentyl) iminodiacetic acid) succinyl] (nickel salt) DGS-NTA(Ni)²⁺ and DGS-NTA (Co)³⁺ were incorporated inside a ghost red blood cell membrane. Successful incorporation of fluorescent lipids was verified by confocal laser scanning microscopy, images are shown in Fig. 1a (incorporation of Atto647-conjugated DOPE lipids) and Fig.1b (incorporation of Atto488-conjugated DOPE lipids). As can be recognized from these images, cell membranes show fluorescence after incorporation of functional lipids.

Further, successful incorporation of DGS-NTA(Co)³⁺ was verified, by subsequently immobilizing recombinant 6x His-tagged green fluorescent protein (GFP) on a surface modified human GRBC in accordance with the present invention, fluorescence microscopy images are shown in Fig.2a. Left column shows bright field images of surface modified human GRBCs (upper image) and unmodified GRBCs together with GFP. Right column shows staining for recombinant 6x His-tagged green fluorescent protein indicated as membrane staining. Membrane staining occurred only when a surface modified cell was used (Ghost + DGS-NTA (Co)³⁺ + GFP), whereas a combination of unmodified cells and the same recombinant 6x His-tagged green fluorescent protein did not result in any surface staining. Accordingly, it can be concluded that surface modified cell according to the present invention, in this example modified with the functional lipid DGS-NTA, can be labeled with GFP.

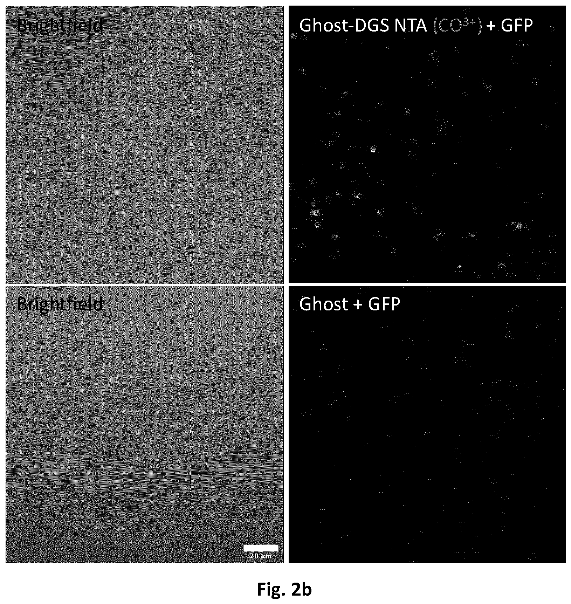

The images shown in Fig. 2b are based on almost the same experiments as the images of Fig. 2a. Instead of human GRBCs, mice GRBCs were functionalized.

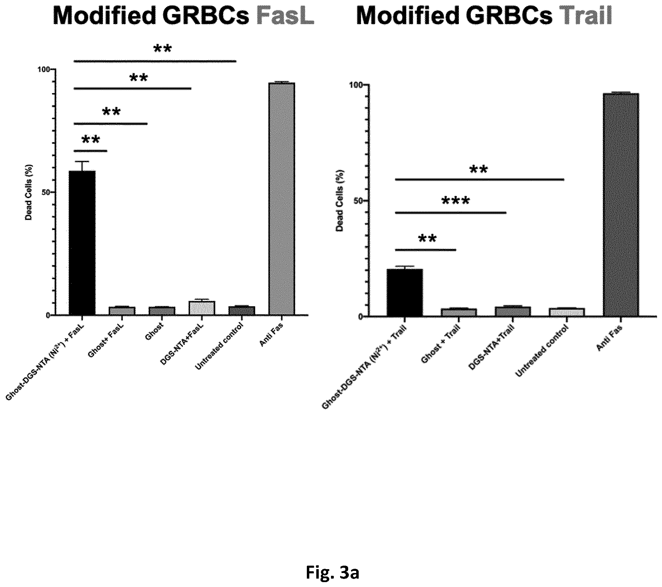

To reconstitute the cytotoxic modules of natural killer cells (NK) and T-cells two recombinant proteins from the tumor necrosis factor (TNF) superfamily, FasL (CD95L or CD 178) and tumor necrosis factor related apoptosis inducing ligand (Trail), were chosen as functional motifs to be attached to a surface modified cell in accordance with the present invention. These ligands are well studied to induce controlled cell death after binding to their receptors via apoptosis through the caspase-8 downstream signaling pathway [15]. Following functionalization of smGRBC surface with these ligands, several bio-assays were implemented to verify their ability to induce apoptosis in target cells. The results are shown in Fig. 3a to Fig. 3d.

Fig. 3a shows a verification of cytotoxic potential of FasL- and Trail-functionalized smGRBCs by incubating them with Jurkat T-cells for 48 h and quantifying propidium iodide (Pi) positive cells by flow cytometry. The FasL used was human FasL (hFasL). It was found, that DGS-NTA modified smGRBCs in accordance with the present invention in combination with immobilized ligands ("Ghost-DGS-NTA (Ni)²⁺+ FasL" in Fig. 3a left side, "Ghost-DGS-NTA (Ni)²⁺+ Trail" in Fig. 3a right side) were able to kill Jurkat T-cells efficiently. To validate the data, the experiment was repeated with only unmodified GRBCs ("Ghost" in Fig. 3a left side), with a combination of unmodified GRBCs and the DGS-NTA + FasL or DGS-NTA + Trail, respectively, recombination protein complex ("Ghost + FasL" in Fig. 3a left side, "Ghost + Trail" in Fig. 3a right side), without any treatment ("untreated control" Fig, 3a left and right side) as negative control and with anti-Fas ("Anti Fas" Fig, 3a left and right side) as positive control. As can be recognized from the Data of Fig. 3a, the smGRBCs having the FasL recombinant protein bound onto their surface is capable of inducing cell death. In contrast, the smGRBCs without any functional motif on their surface and the unmodified GRBCs, did not show any significant induction of cell death. Also, the DGS-NTA-FasL as such did not induce cell death. Corresponding observations were made for the Trail-functionalized smGRBCs and the corresponding control experiments (see Fig. 3a right side). The data allows the conclusion that in order to induce cell death, it is required that the FasL or Trail ligands are bound to the surface of the GRBCs. The unmodified GRBCs alone, in combination with DGS-NTA + FasL or DGS-NTA + Trail (not incorporated into the cell membrane) did not show any significant difference to the untreated control.

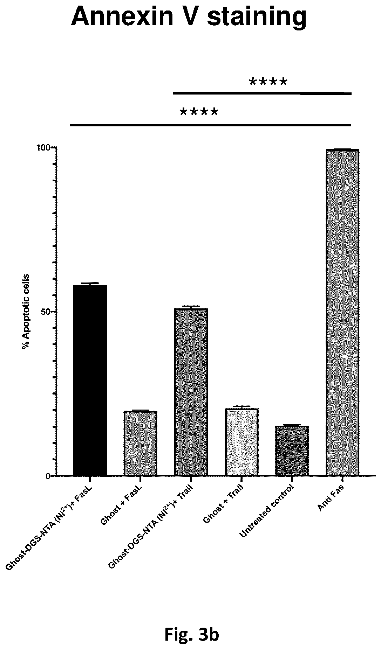

The smGRBCs of the present invention functionalized with recombinant proteins FasL or Trail are capable of inducing the controlled cell death in Jurkat T-cells *via* apoptosis. The data shown in Fig. 3b was obtained from incubating the FasL or Trail functionalized smGRBCs with Jurkat T-cells for 24 h and subsequently staining the membrane with Annexin V, an early apoptosis indicator. Afterwards, the percentage of Annexin V positive cells were quantified by flow cytometry and termed as apoptotic positive cells (Figure 3b). From the Annexin V quantification we obtained comparable results as PI quantification revealing that only smGRBCs functionalized with bio active ligands are able to induce apoptosis in Jurkat T-cells.

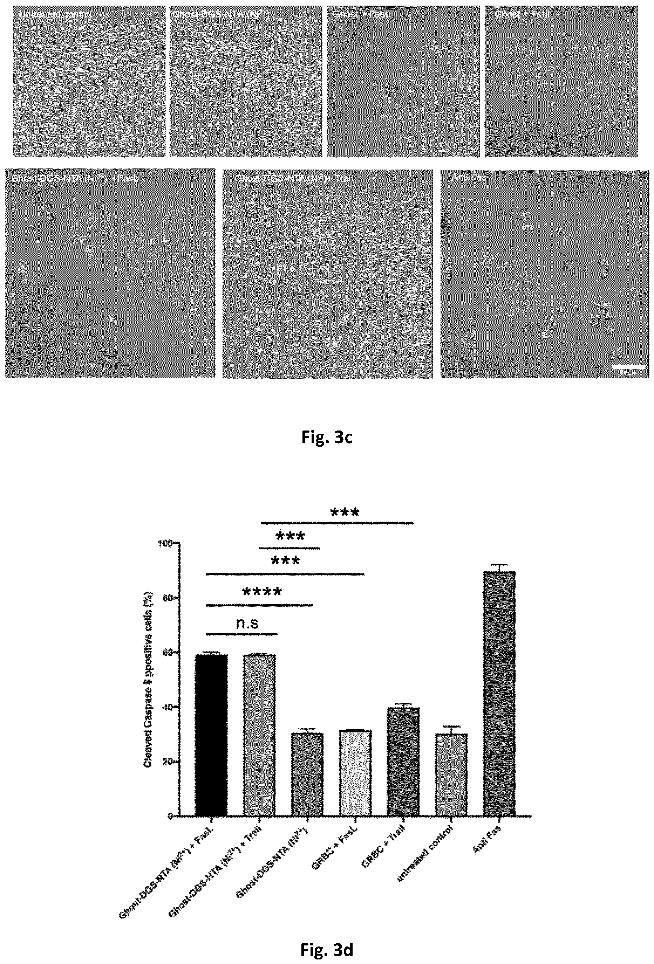

In addition to the early apoptosis indicator Annexin V, it is shown by the data of Fig. 3c and Fig. 3d that down-stream the apoptosis signaling cascade, caspase-8 is activated. To obtain the data shown in Fig. 3c and Fig. 3d, the smGRBCs were incubated with Jurkat T-cells for 24 h and cleaved caspase-8 positive cells were identified through immunocytochemistry (Figure 3c). Further flow cytometry to quantify the percentage of cleaved caspase-8 positive cells (Figure 3d). Taken these results together we verified that smGRBCs with FasL/Trail induce apoptosis through caspase-8 downstream signaling similarly to their immunological blueprints.

The following is a more detailed figure labeling for Figures 3a to 3d. Cytotoxic activity of human smGRBC functionalized with FasL orTrail. Fig. 3a) Flow cytometry quantification of PI positive Jurkat T-cells incubated with FasL- or Trail-functionalized smGRBC, unmodified GRBC with soluble FasL or Trail, GRBC, DGS-NTA with FasL or Trail, untreated control and Anti Fas as positive control. Mean+/- SD, n=3 and unpaired t-test with Welch's correction ***p < 0.0005, ****p.<o.00005 are presented. Fig. 3b) Flow cytometric quantification of Annexin V positive Jurkat T-cells incubated with smGRBC with FasL, unmodified GRBCs with FasL, smGRBC with Trail, unmodified GRBC with Trail, untreated control and Anit Fas as positive control. Mean+/- SD, n=3 and unpaired t-test with Welch's correction **p < 0.005, ***p<0.0005 are presented. Fig. 3c) Anti-cleaved caspase-8 staining in Jurkat T-cells treated with different conditions. Upper panel from left to right: left untreated (untreated control), incubated with smGRBC, unmodified GRBC with FasL and unmodified GRBC with Trail. Lower panel from left to right: smGRBC with FasL, smGRBCs with Trail and Anti Fas as positive control. Fig. 3d) Flow cytometric quantification of anti-cleaved caspase-8 positive Jurkat T-cells incubated with smGRBC with FasL, smGRBC with Trail modified GRBC, unmodified GRBC with FasL unmodified GRBCs with Trail, untreated control and Anti Fas as positive control. Mean+/- SD, n=3 is shown and unpaired t-test with Welch's correction ***p <0.0005, ****p.<0.00005 are presented.

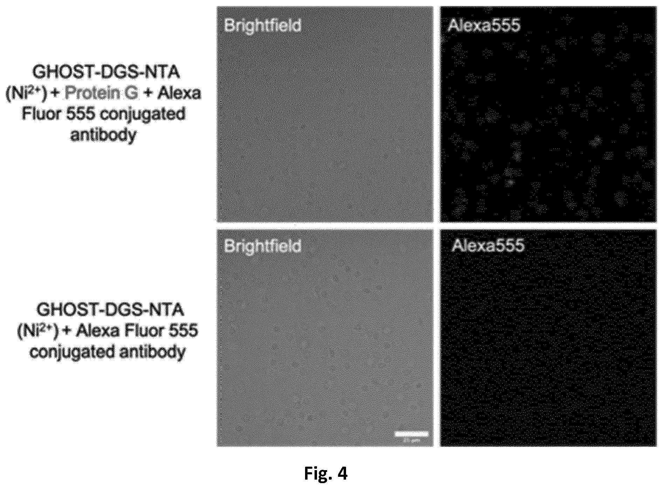

In order to provide the smGRBC with the specificity of B-cells a sequential immobilization process of Fc-receptors and immunoglobulins was performed. Immobilization of Fc-receptors (Protein G) and immunoglobulins conjugated to an Alexa555 fluorophore was verified by confocal laser scanning microscopy (Figure 4).

The left column of the images of Fig. 4 shows bright field images of smGRBCs. The right column shows fluorescent signal of AlexaFluor 555 conjugates antibody in presence and absence of binding-mediated Protein G.

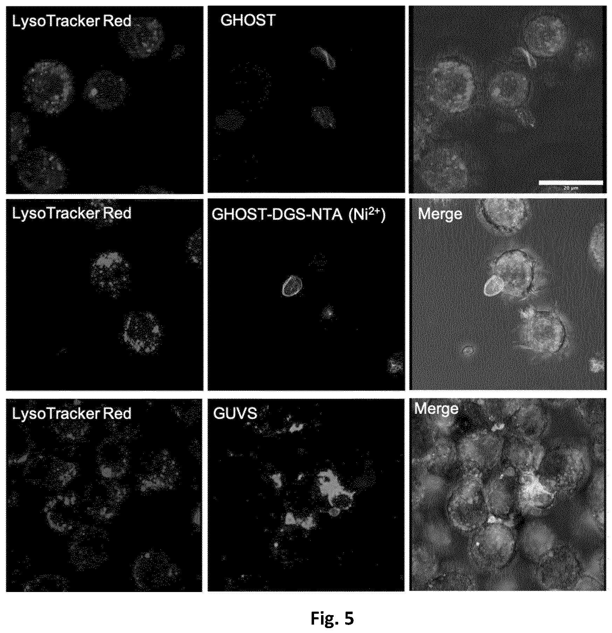

In order to test that smGRBCs are not taken up by innate immune cells smGRBCs were incubated with WBC264-9C macrophages. Unmodified GRBC ("GHOST" in Fig. 5) and surface modified ghost red blood cells (smGRBCs) ("GHOST-DGS-NTA" in Fig. 5) in accordance with the present invention were stained with an Alexa488 amine reactive dye and incubated them with WBC264-9C macrophages. As suggested by the labeling "GHOST-DGS-NTA" of Fig. 5, the smGRBCs were modified by incorporating the functional lipid DGS-NTA (Ni²⁺)into the cell membrane. The lysosomes of macrophages, the major degradation compartment of cells, were stained to assess if a colocalization of the signal form smGRBCs and lysosomes can be detected. It could be verified that no co-localization of the smGRBC signal and the lysosomes can be detected, indicating that the smGRBC are not up taken by macrophages (Figure 5). As a control, giant unilamellar vesicles (GUVS) ("GUVS" in Fig. 5) harboring Atto488 flourescent tagged lipids were incubated with WBC264-9C macrophages. It can be recognized that the GUVS were taken up by the macrophages by the colocalization of the Atto488-fluorescence signal and lysosomes of the macrophages.

The following is a more detailed figure labeling for Figure 5. GRBC, smGRBC and GUVs interaction with WBC264-9C macrophages. Fluorescent confocal microscopy images of WBC264-9C macrophages stained with LysoTracker red (lysosomes, red) incubated with Alexa488-TFP stained GRBCs or smGRBCs or GUVS harboring Atto488 conjugated lipids (plasma membrane, green).

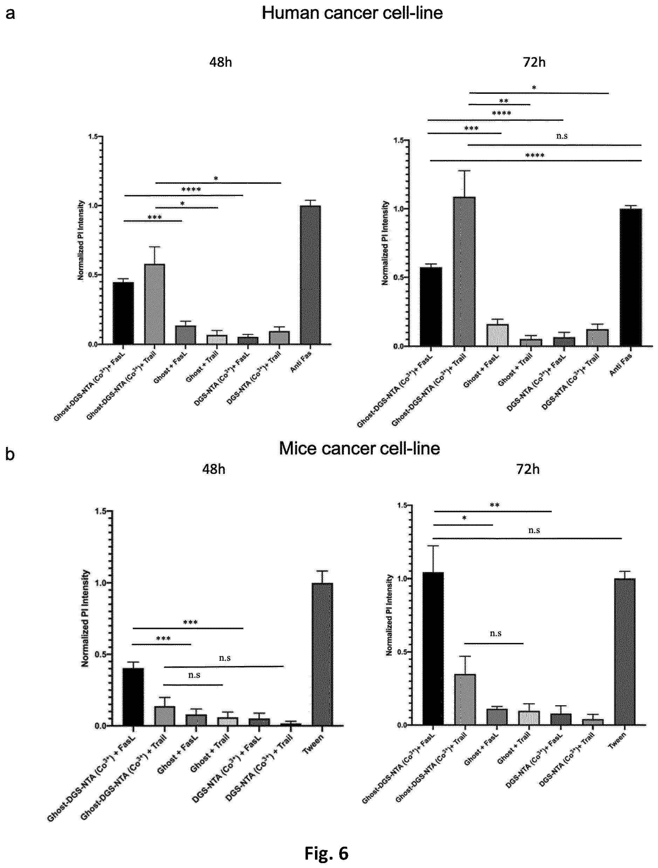

Similar to the experimental results shown in of Fig. 3a the cytotoxicity of other modified cells in accordance with the present invention was examined. The results are shown in Fig. 6a (cytotoxicity on a human cancer cell-line) and in Fig. 6b (cytotoxicity on a human cancer cell-line) (cytotoxicity on a human cancer cell-line).

The data shown in Fig. 6a and Fig. 6b was obtained from mice smGRBC immobilized with cytotoxic ligands. The smGRBC and the adducts with FasL and Trail were obtained as described by the corresponding method below. Fig. 6a shows the cytotoxic effect of the surface modified cells according to the present invention on a human cancer cell-line by showing the PI intensity quantification, i.e. the cytotoxic effect on Jurkat T-cells incubated for 48h or 72h with human FasL mice smGRBC (Co³⁺ complexed), Trail mice smGRBC (Co³⁺complexed) unmodified mice GRBC incubated with FasL, or Trail (not according to the invention), unmodified GRBC (not according to the invention), DGS-NTA (Co³⁺ complexed) with FasL, or Trail (not according to the invention) and Anti Fas as positive control (not according to the invention). Mice GRBCs functionalized with FasL and with Trail show cytotoxic effects on Jurkat T-cells already after an incubation of 48h. The effect increased after 72h to a degree that the functionalization with Trail did not show a significant difference to the positive control. The statistics of Fig. 6 a is as follows: Mean+/- SD, n=3 is show. Unpaired t-test with Welch's correction *p < 0.05, **p < 0.005, ***p < 0.0005, ****p<0.00005, n.s not significant.

In addition to the examination of cytotoxicity of the modified cells of the present invention after functionalization with FasL or Trail on a human cancer cell-line, the cytotoxicity on a mice cancer cell-line was examined. The results are shown in Fig. 6b in form of PI intensity quantification of A.20 B-cell lymphoma cells after incubated for 48h or 72h with human FasL mice smGRBC (Co³⁺ complexed), Trail mice smGRBC (Co³⁺ complexed), unmodified mice GRBC with soluble FasL (not according to the invention), or with soluble Trail (not according to the invention), unmodified GRBC (not according to the invention), DGS-NTA (Co³⁺ complexed) incubated with FasL (not according to the invention), or Trail (not according to the invention) and Anti Fas (not according to the invention) and Tween positive control (not according to the invention). After 48 hours of incubation already an effect of the surface modified cells functionalized with FasL could be observed. Notably, FasL has an effect on a mice cancer cell-line. After 72h, there was no significant difference between the effect of surface modified cells functionalized with FasL and the Tween positive control.

The statistics of Fig. 6b is as follows: Mean+/- SD, n=3 is show. Unpaired t-test with Welch's correction *p < 0.05, **p < 0.005, ***p < 0.0005, n.s not significant.

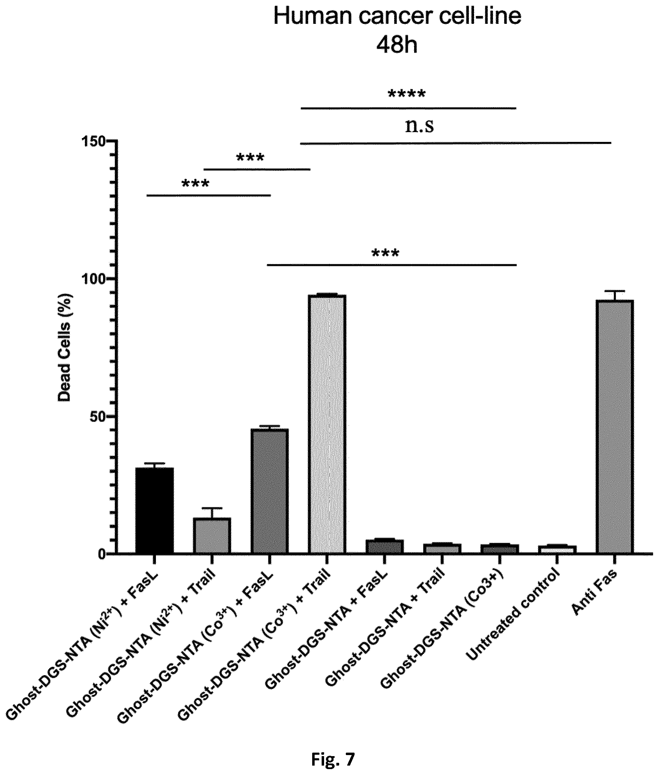

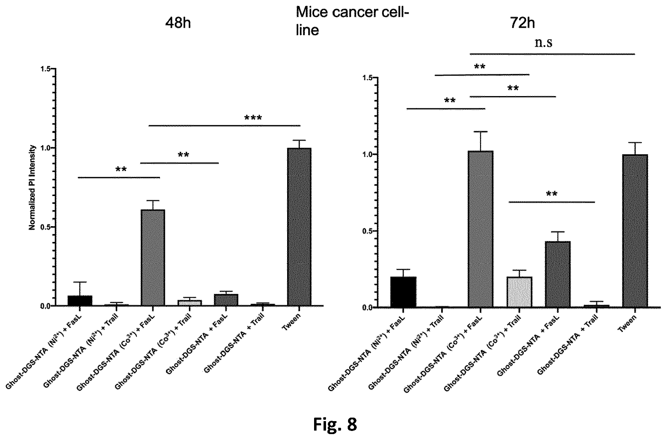

To further investigate the effect of the immobilization of the functional motif on the surface of the modified cell, the data shown in Fig. 7 and in Fig. 8 was prepared. The data of Fig. 7 was obtained similar to that of Fig. 6a, however instead of PI intensities, the percentage of dead cells as determined by flow cytometric quantification of PI positive Jurkat T-cells is shown. Jurkat T-cells were incubated with smGRBC functionalized with human FasL/Trail (Co³⁺or Ni²⁺ complexed), smGRBC un-complexed with human FasL or Trail (not according to the invention), smGRBC (Co³⁺ complexed) and Anti Fas as positive control (not according to the invention). The complexation with Co³⁺ and with Ni²⁺ is suitable for providing the surface modified cells of the present invention with cytotoxic properties. These properties were stronger for complexation with Co³⁺ compared to complexation with Ni²⁺. It is assumed that this is due to the lower metal exchange rate for the Co³⁺-complexes in comparison to Ni²⁺-complexes.
statistics of Fig. 7 is as follows: Mean+/- SD, n=3 is show. Unpaired t-test with Welch's correction ***p < 0.0005, n.s not significant.

### Preparation of human ghost red blood cells (unmodified GRBCs)

In order to obtain human ghost red blood cells from erythrocyte concentrate, 600 µl of red blood cells where mixed together with 800µl 1X PBS (phosphate buffered saline) supplemented with 0.3 mM Phenylmethansulfonylfluorid (PMSF) and centrifuged at 4000g at 4°C for 10 minutes. Afterwards, the supernatant was discarded and pallets were resuspended in 1X PBS supplemented with 0.3 mM PMSF and centrifugation was repeated one mor time. Next, the supernatant was discarded and pellet was resuspended in a hypotonic buffer (1X PBS mixed with ddH2O in a ratio of 1:31.25, pH 8.0 and supplemented with 0.3 mM PMSF) and set incubated for 1 hour on ice. Subsequently, the sample was pelleted at 20'000g and 4°C for 15 min and the supernatant was discarded. After repeating this process four times the pellet was resuspended in 1X PBS supplemented with 0.3 mM PMSF and pelleted at 20'000g at 4°C for 15 min. Finally, the pellet was washed twice with the hypotonic buffer.

### Preparation of mice ghost red blood cells (unmodified GRBCs)

In order to obtain mice ghost red blood cells from whole blood, 700 µl of whole blood centrifuged at 4'000g at 4°C for 30 min. Afterwards, the supernatant was discarded and the pellet was resuspended in 800 µl 1X PBS supplemented with 0.3 mM Phenylmethansulfonylfluorid (PMSF) and centrifuged at 4000g at 4°C for 30 minutes. Afterwards, the supernatant was discarded and the pellet was resuspended in 1X PBS supplemented with 0.3 mM PMSF and pelleted at 4'000g at 4°C for 15 min. This process was repeated 2 times. Subsequently, the supernatant was discarded and the pellet was resuspended in a hypotonic buffer (1X PBS mixed with ddH2O in a ratio of 1:31.25, pH 8.0 and supplemented with 0.3mM PMSF) and set incubated for 1 hour on ice. Subsequently, the sample was pelleted at 20'000g and 4°C for 15 min and the supernatant was discarded. After repeating this process four times the obtained pellet was resuspended in 1X PBS supplemented with 0.3 mM PMSF and pelleted at 20'000g at 4°C for 15 min. Finally, the pellet was washed twice with the hypotonic buffer.

### Preparation of surface modified ghost red blood cells (smGRBCs) and bonding of functional motifs

For the incorporation of functional lipids fatty acid free bovine serum (BSA) was dissolved in 1X PBS to a concentration of 13.6 mg/ml and vortexed for 30 s. All functional lipids were stored in chloroform at -20°C. The lipid solvent was pipetted in glass vials and subsequently dried under vacuum for at least 15 min to evaporate the chloroform completely. Subsequently, 5 µl of 100% Ethanol (EtOH) was added to the obtained lipid film and rehydrated with the fatty acid free BSA solution. To the lipid fatty acid free BSA EtOH mixture 2.25 ml of 1XPBS was added and vortexed for 30 s leading to a concentration of 1.36 mg/ml fatty acid free BSA and a lipid concentration of 0.03 mg/ml.

In order to incorporate the lipids inside the GRBC membrane 600 µl of the above-described mixture was added to a 1.4 ml 1XPBS solution containing the GRBCs with the desired amount, leading to a final concentration of 0.59mg/ml fatty acid free BSA and 0.013 mg/ml functional lipids. Subsequently, the solution was incubated for 30 min at 37°C under constant shaking (800 rpm) in an Eppendorf ThermoMixer. Afterwards, the functional lipid incorporated GRBCs were pelleted at 10'000g for 10 min at RT. The supernatant was discarded and the pellet was dissolved in 1ml 1XPBS or in a 1 M NaHCO3 solution containing 1 mM of Na₃ [Co (III) (Co₃)₃]*3H₂O. The lipid incorporated GRBCs dissolved in 1 M NaHCO₃ solution containing 1mM of Na3 [Co (III) (Co₃)₃]*3H₂O was further set incubated for 10 min, 37°C and constant shaking (800 rpm) in an Eppendorf ThermoMixer. In order to get rid of unbound Co³⁺ the mixture was washed two times in 1XPBS by centrifugating at 10'000g for 10 min at RT. Finally, the pallet was dissolved in 1 ml 1XPBS. For the bio-orthogonal functionalization of the lipid incorporated GRBCs with recombinant proteins 100 µl of the final mixture was taken and incubated with the desired proteins. To this end, 6x His-tagged GFP, 8x His-tagged FasL, poly His-tagged Trail and 6x His-tagged Protein G were added at a concentration of 0,01 mg/ml, 0.002 mg/ml, 0.002 mg/ml and 0.004 mg/ml, respectively and set incubated for 30min at RT. Subsequently, the smGRBCs were pelleted at 10'000g for 10 min at RT. Finally, the supernatant was discarded and the pellet was resuspended in 50 µL PBS.

### Bonding of functional motifs to smGRBCs

### References:

[1] Subramanian, N., et al., Network representations of immune system complexity. Wiley Interdiscip Rev Syst Biol Med, 2015. 7(1): p. 13-38.
[2] Kim, S.K. and S.W. Cho, The Evasion Mechanisms of Cancer Immunity and Drug Intervention in the Tumor Microenvironment. Frontiers in Pharmacology, 2022. 13**.**
[3] Rafiq, S., C.S. Hackett, and R.J. Brentjens, Engineering strategies to overcome the current roadblocks in CAR T cell therapy. Nature Reviews Clinical Oncology, 2020. 17(3): p. 147-167.
[4] Li, D., et al., Genetically engineered T cells for cancer immunotherapy. Signal Transduct Target Ther, 2019. 4: p. 35.
[5] Kandra, P., et al., Utility and Drawbacks of Chimeric Antigen Receptor T Cell (CAR-T) Therapy in Lung Cancer. Frontiers in Immunology, 2022. 13**.**
[6] Hernandez Bücher, J.E., et al., Bottom-up assembly of target-specific cytotoxic synthetic cells. Biomaterials, 2022. 285: p. 121522.
[7] Szebeni, J. and S.M. Moghimi, Liposome triggering of innate immune responses: A perspective on benefits and adverse reactions. Journal of Liposome Research, 2009. 19(2): p. 85-90.
[8] Tang, Y., et al., Overcoming the Reticuloendothelial System Barrier to Drug Delivery with a "Don't-Eat-Us" Strategy. ACS Nano, 2019. 13(11): p. 13015-13026.
[9] Dammes, N., et al., Conformation-sensitive targeting of lipid nanoparticles for RNA therapeutics. Nat Nanotechnol, 2021. 16(9): p. 1030-1038.
[10] Gómez Flares, V., et al., Biointeraction of Erythrocyte Ghost Membranes with Gold Nanoparticles Fluorescents. Materia Is (Basel), 2021. 14(21).
[11] Franco, R.S., Measurement of red cell lifespan and aging. Transfus Med Hemother, 2012. 39(5): p. 302-7.
[12] Himbert, S., et at., Hybrid Erythrocyte Liposomes: Functionalized Red Blood Cell Membranes for Molecule Encapsulation. Advanced Biosystems, 2020. 4(3): p. 1900185.
[13] Guo, J., et al., Biomimetic Rebuilding of Multifunctional Red Blood Cells: Modular Design Using Functional Components. ACS Na no, 2020. 14(7): p. 7847-7859.
[14] Rossi, L., et al., Red Blood Cell Membrane Processing for Biomedical Applications. Frontiers in Physiology, 2019. 10.
[15] Knight, M.J., et al., Analysis of FasL and TRAIL induced apoptosis pathways in glioma cells. Oncogene, 2001. 20(41): p. 5789-98.

## Claims

1. Modified cell, in particular a modified blood cell, having a cell membrane encapsulating a lumen of the modified cell,
wherein a functional lipid is incorporated into the cell membrane,
wherein the functional lipid has a bonding site capable of bonding to a molecule having a functional motif, and
wherein the bonding site is located on an external surface of the cell membrane.

2. Modified cell according to claim 1,
wherein the modified cell is a surface modified natural cell or a surface modified ghost natural cell.

3. Modified cell according to claim 2, wherein the modified cell is a modified blood cell, in particular a red blood cell, in particular a modified ghost red blood cell.

4. Modified cell according to any one of claims 1 to 3,
wherein the functional lipid is bonded to a molecule having a functional motif via the bonding site,
preferably wherein the functional lipid is bonded to the molecule having the functional motif by a covalent bond or by a complex formation.

5. Modified cell according to claim 4,
wherein the bond between the functional lipid and the molecule having the functional motif is obtained by formation of a complex or by at least one covalent bond in particular by at least one covalent bond formed by a click chemistry reaction.

6. Modified cell according to any one of claims 4 or 5,
wherein the bond between the functional lipid and the functional motif is obtained by formation of a complex, wherein the complex comprises
(i) a metal cation,
(ii) the bonding site of the functional lipid, and
(iii) the molecule with the functional motif,
wherein the bonding site of the functional lipid and the molecule with the functional motif are ligands to the metal cation.

7. Modified cell according to claim 6,
wherein the bonding site of the functional lipid is a chelating ligand.

8. Modified cell according to claim 6 or 7,
wherein the bonding site of the functional lipid has a chemical structure derived from nitrilotriacetic acid (NTA), iminodiacetic acid (IDA), tris(carboxymethyl)ethylenediamine (TED), triazacyclononane (TACN), diethylenetriamine-pentaacetate (DTPA), phytochelatin, carboxymethylaspartate (CMA), phosphonates, tannic acid (TA), porphyrin, dipyridylamine (DPA), phytic acid, nitrilopropionicdiacetic acid (NPDA), nitriloisopropionicdiacetic acid (NIPDA), N-(hydroxylethyl)ethylenediaminetriacetic acid (HEDTA), 1,4,7,10-tetraazacyclodo-decane-N,N',N",N‴-tetraacetic acid (DOTA), 1,4,7-tris(carboxymethyl)-10-(2'-hydroxypropyl)-1,4,7,10-tetraazocyclodecane, 1,4,7-triazacyclonane-1,4,7-triacetic acid (NOTA), 1-(1,3-carboxypropyl)-1,4,7-triazacyclononane-4.7-diacetic acid (NODAGA), 1,4,8,11 -tetraazacyclo-tetra-decane- N,N',N",N‴-tetraacetic acid (TETA), ethylenedicysteine, ethylenediaminetetraacetic acid (EDTA), 1,2-diaminocyclohexane-N,N,N',N'-tetraacetic acid (DACT), bis(aminoethanethiol)carboxylic acid, ethylene-bis(oxyethylene-nitrilo)tetraacetic acid (EGTA), triethylenetetramine-hexaacetic acid (TTHA), 1,4,7-triazacyclononane phosphinic acid (TRAP), deoxyribonucleic acid (DNA) and ribonucleic acid (RNA), purine, and pyridimidine and derivatives thereof,
preferably wherein the bonding site of the functional lipid has a chemical structure derived from nitrilotriacetic acid (NTA), iminodiacetic acid (IDA), chelating peptides with the consensus sequence (GHHPH) n G, diethylenetriamine- pentaacetate (DTPA), nitrilopropionicdiacetic acid (NPDA), nitriloisopropionicdiacetic acid (NIPDA), ethylenediamine-tetraacetic acid (EDTA), ethylene-bis(oxyethylene- nitrilo)tetraacetic acid (EGTA), carboxymethylaspartate (CMA) and derivatives thereof,
more preferably wherein the bonding site of the functional lipid has a chemical structure derived from NTA, IDA and derivatives thereof.

9. Modified cell according to any one of claims 6 to 8, wherein the metal cation of the complex is a di-, tri- or tetravalent metal cation.

10. Modified cell according to any one of claims 6 to 9, wherein the metal cation is selected from the group consisting of: Ni²⁺, Co³⁺, Cr³⁺, Rh³⁺, Ir³⁺, Pt²⁺, Pt⁴⁺, Ru²⁺, Ru³⁺, La³⁺, Eu³⁺, Os²⁺, Pd⁴⁺, Mo³⁺, Fe³⁺, Ru³⁺, Gd³⁺, Tc³⁺, Re³⁺, Sm³⁺, Tb³⁺, Ce³⁺, Pr³⁺, Nd³⁺, Pm³⁺, Dy³⁺, Ho³⁺, Er³⁺, Tm³⁺, Yb³⁺, V²⁺, Mn⁴⁺, Fe²⁺ and Lu³⁺, preferably wherein the metal cation is selected from the group consisting of: Ni²⁺, Co³⁺, Cr³⁺, Rh³⁺, Ir³⁺, Ir⁴⁺, Pt²⁺, Pt⁴⁺, Pd⁴⁺, Mo³⁺, Fe³⁺, Gd³⁺, Tb³⁺, Eu³⁺, Ru²⁺, La³⁺, Ru³⁺, Re³⁺, Re⁴⁺, V²⁺, Mn⁴⁺, Fe²⁺ and Os²⁺, more preferably wherein the metal cation is Ni²⁺ or Co³⁺, even more preferably wherein the metal cation is Co³⁺.

11. Modified cell according to any one of claims 6 to 10,
wherein the molecule having the functional motif has an affinity tag, in particular a His-tag having six to nine histidine moieties, and
wherein the affinity tag is a chelate ligand to the metal cation.

12. Modified cell according to any one of claims 1 to 11,
wherein the functional lipid is a polar lipid, in particular a phospholipid or cholesterol.

13. Modified cell according to any one of claims 1 to 12,
wherein the functional motif is based on a protein, in particular wherein the molecule with the functional molecule is a recombinant protein combining the functionality of the protein and is provided with a site to bond to the bonding site of the functional lipid.

14. Modified cell according to claim 13,
wherein the functional motif is based on a protein from the tumor necrosis factor superfamily,
in particular wherein the functional motif is at least one of FasL, in particular human FasL, and Trail.

15. Modified cell according to any one of claims 1 to 14,
wherein the functional motif is a label, in particular a fluorescence label, radioactive label, or a magnetic label.

16. Pharmaceutical composition comprising a modified cell according to any one of claims 1 to 15.

17. Modified cell according to any one of claims 1 to 15 or pharmaceutical composition according to claim 22 for use as a medicament or as a contrast agent, in particular for imaging technology such as diagnostic imaging.

18. Modified cell or pharmaceutical composition for use according to claim 17, wherein the medicament or contrast agent is administered by injection, in particular by injection into a vein,
preferably wherein the medicament is for use in the treatment of a disease, more preferably wherein the disease is a tumor disease, a cardiovascular disease, a neurodegenerative disease, an autoimmune disease, or an inflammation disease.

19. Method of modifying a cell,
wherein the method comprises the steps of
(A) providing a target cell to be modified,
(B) incorporating a functional lipid into the cell membrane of the target cell, wherein the functional lipid has a bonding site, and
(C) linking a functional motif to the binding site of the lipid.

20. Method according to claim 19,
wherein the cell obtained by the method is a modified cell according to any one of claims 1 to 15.
